Europäisches Patentamt

(19) European Patent Office

Office européen des brevets

(11) Publication number: **0 005 378**
**B1**

(12) **EUROPEAN PATENT SPECIFICATION**

(45) Date of publication of patent specification: **24.02.82**

(51) Int. Cl.³: **C 07 C 17/10, C 07 C 19/02**

(21) Application number: **79300790.7**

(22) Date of filing: **09.05.79**

(54) Chlorination of hydrocarbons.

(30) Priority: **10.05.78 ZA 782685**

(43) Date of publication of application:
**14.11.79 Bulletin 79/23**

(45) Publication of the grant of the patent:
**24.02.82 Bulletin 82/8**

(84) Designated Contracting States:
**CH DE FR GB IT NL**

(56) References cited:
**DE - A - 706 197**
**FR - A - 1 196 916**
**FR - A - 1 505 763**
**FR - A - 1 508 460**
**US - A - 2 941 013**
**US - A - 3 806 320**

(73) Proprietor: **KLIPFONTEIN ORGANIC PRODUCTS CORPORATION LIMITED**
**Siding No.1423**
**Elandsfontein Transvaal (ZA)**

(72) Inventor: **Gilliland, John Barry**
**27 Nile Street Kensington**
**Johannesburg Transvaal (ZA)**
Inventor: **Morgan, David Lewis**
**106 Longridge Ridge Road Glenhazel**
**Johannesburg Transvaal (ZA)**

(74) Representative: **Crawford, Andrew Birkby**
**A.A. THORNTON & CO. Northumberland House**
**303-306 High Holborn**
**London WC1V 7LE (GB)**

Courier Press, Leamington Spa, England.

Chlorination of hydrocarbons

This invention relates to the chlorination of hydrocarbons, particularly paraffin hydrocarbons.

The chlorination of paraffin hydrocarbons is generally carried out batchwise in the liquid phase in agitated reactor vessels. The reaction, which is initiated by free radicals, may be chemically, thermally, or photochemically catalysed. The agitation is generally mechanical, and vessels of a relatively large volume are employed.

These known batch processes have a number of disadvantages including low efficiency of mass transfer of chlorine gas into the liquid phase and long periods of initiation of the chlorination reaction. They accordingly suffer from low throughputs per unit of reactor volume and high chlorine losses. Another disadvantage is that to obtain a stable chlorinated paraffin hydrocarbon of high quality, a straight chain paraffin feedstock of very high purity must be used. It is often difficult to obtain such a feedstock.

We have now developed an improved process for the batchwise chlorination of hydrocarbons whereby improved mass transfer of chlorine gas into the liquid phase and highly efficient use of the reactor volume are obtained.

According to the present invention, there is provided a batch process for chlorinating a hydrocarbon which is a liquid hydrocarbon or a solid hydrocarbon suspended in a suitable liquid, which comprises:

(a) reacting a charge of the hydrocarbon with chlorine in a vertically disposed column reactor having a reactor volume in which the height is at least four times the longest transverse dimension, to obtain a chlorinated hydrocarbon and residual reaction gases;

(b) stripping a second charge of the hydrocarbon with the residual reaction gases produced in step (a) in order to remove from the second charge inhibitors of chlorine free radical formation;

(c) discharging the chlorinated hydrocarbon produced in step (a) when a predetermined degree of chlorination has been attained; and

(d) re-charging the column reactor with the stripped second charge of hydrocarbon produced in step (b).

Column reactors, as used in the process according to the invention, are well known in the art. As indicated, such reactors are vertically disposed and have a reactor volume wherein the height is at least four times the longest transverse dimension, which is generally the diameter as such reactors are generally cylindrical.

The extent of chlorination effected in the process will depend upon the type and nature of chlorinated hydrocarbon which is desired. The chlorination proceeds through free radical initiation in the conventional manner.

The hydrocarbon charge is preferably continuously circulated through the column while chlorine gas is continuously fed thereto. Preferably the charge and the chlorine gas are caused to flow countercurrently, the charge downwardly and the gas upwardly through the reactor and the charge being bled from a low point of the reactor and recirculated to a high point of the reactor, and residual reaction gases being removed from a high point of the reactor.

The stripping of the second charge is preferably effected in a second column reactor similar to that used for step (a) and in which the second charge and the residual reaction gases are caused to flow countercurrently, the second charge downwardly and the gases upwardly, the second charge being discharged from a low point of the second reactor and recirculated to a high point thereof, and the residual reaction gases being removed from a high point of the second reactor.

The use of the residual reaction gases from step (a) to strip the second charge has a number of advantages. First, stripping of the inhibitors is effectively achieved. Second, any chlorine in the reaction gases reacts with the charge so that partial chlorination of the charge is achieved in the stripping stage. This chlorination assists in maintaining the charge at an elevated temperature. When there is thermal initiation of the reaction, then the charge is preferably introduced into the column reactor at a temperature exceeding 50°C. The charge in these circumstances is typically introduced at a temperature in the range 70°C to 90°C. When there is photochemical initiation, lower charge temperatures achieve very satisfactory results.

The reaction gases typically contain 0 to 15% molecular chlorine, with the balance being mainly hydrogen chloride.

The contents of the chlorination reactor are generally maintained at a temperature exceeding 80°C, preferably in the range 110°C to 130°C. Since the chlorination reaction is exothermic, cooling will generally be required to maintain the temperature in the preferred range.

The hydrocarbon charge may be a liquid hydrocarbon such as toluene or a paraffin hydrocarbon or a solid hydrocarbon suspended in a suitable liquid, e.g. solid polyethylene in carbon tetrachloride. The paraffin hydrocarbon charge may be, for example, one consisting of a mixture of essentially straight-chain paraffins having an average chain length between 9 and 26 carbon atoms. Chlorination of paraffin hydrocarbons will generally be continued until chlorinated paraffins having a chlorine content up to 76% by weight are produced.

The process in its preferred form may be carried out in apparatus comprising a stripping column reactor, a chlorinating column reactor, means for feeding a hydrocarbon charge from the stripping reactor to the chlorinating reactor, means for continuously recirculating the hydrocarbon charge through each of the column reactors, means for feeding chlorine gas to the chlorinating reactor, means for discharging chlorinated hydrocarbon charge from the chlorinating reactor, means for bleeding residual reaction gases from the chlorinating reactor and feeding them into the stripping reactor, means for bleeding residual reaction gases from the stripping reactor, and means for regulating the temperatures in the stripping reactor and chlorinating reactor.

The column reactors may be of any suitable material known in the art, but are preferably of glass.

The dimensions of the column reactor for the chlorination reaction may be selected according to the particular application, the relationship between height and longest transverse dimension preferably being selected to provide for the optimum gas-liquid contact in the column. The ratio of reactor volume height to longest transverse dimension will be as described above.

The temperature-regulating means may be heat-exchange coils or like heat-exchange systems.

The invention is discussed further with reference to the accompanying flowsheet which illustrates one embodiment of the invention.

Referring to the flowsheet, a system for the batch chlorination of a liquid hydrocarbon comprises a stripping column reactor 2 and a pair of chlorination columns 4, 6.

The stripping column reactor 2 has a gas outlet pipe 8 at its top, a hydrocarbon inlet pipe 10 and a gas inlet pipe 12 near its foot, and at its foot a hydrocarbon outlet pipe 14 connected to a pump 16 connected to a recirculation pipe 18 which leads into the stripping column reactor 2 near its top.

A hydrocarbon discharge pipe 20 leads from the recirculation pipe 18 and branches into two separate hydrocarbon feed pipes 22, 24 leading into the lower ends of the column reactors 4, 6.

Each column reactor 4, 6 has a gas inlet pipe 26, 28 near its lower end, and a gas outlet pipe 30, 32 at its top, the gas outlet pipes 30, 32 leading into the gas inlet pipe 12 of the stripping column reactor 2. Each column reactor 4, 6 also has at its foot a hydrocarbon outlet pipe 34, 36 connected to a pump 38, 40 connected to a recirculation pipe 42, 44 which leads into the column reactor 4, 6 near its top.

A chlorinated hydrocarbon discharge pipe 46, 48 leads from each recirculation pipe 42, 44 into a common discharge pipe 50.

The stripping column reactor 2 and the chlorination column reactors 4, 6 are made of glass.

The system operates as follows. Hydrocarbon feedstock is charged to the stripping column reactor 2 through the inlet pipe 10 to fill the reactor 2. Stripping is effected by feeding residual reaction gases from the chlorination reactors 4, 6 into the stripping reactor 2 through the gas inlet pipe 12. The hydrocarbon feedstock in the reactor 2 is withdrawn from the reactor 2 through the outlet pipe 14, and recirculated to the reactor 2 through the recirculation pipe 18 by the pump 16. The gases fed to the reactor 2 are withdrawn through the gas outlet pipe 8 and fed to a conventional absorber (not shown).

The stripping gas is largely hydrogen chloride, but does contain some chlorine. As the stripping gas passes up the reactor 2 it removes traces of free radial inhibitors such as dissolved oxygen from the hydrocarbon feedstock, while residual chlorine reacts with the hydrocarbon feedstock.

The temperature in the stripping column 2 is maintained in the range 70°—90°C by a cooling coil 13. Cooling can also be achieved by locating a suitable heat exchanger in the pipe 18.

The chlorination reactors 4, 6 are charged with predetermined amounts of stripped hydrocarbon feedstock by withdrawing these from the stripping column reactor 2 along the discharge pipe 20 and feeding them to the reactor columns 4, 6 through the inlet pipes 22, 24. Chlorine gas is fed to the reactors 4, 6 through the gas inlet pipes 26, 28 and the hydrocarbon charge in each reactor 4, 6 is circulated through each reactor 4, 6 from top to bottom by means of the pumps 38, 40, the hydrocarbons being withdrawn from the bottom of each column 4, 6 through the outlet pipes 34, 36 and returned to the columns near their tops through the recirculation pipes 42, 44.

The chlorine gas as it passes up each column 4, 6 reacts with the hydrocarbons and when it reaches the top of the column 4, 6 residual gas is led away through the gas outlet pipes 30, 32 and fed to the stripping column reactor 2 through the gas pipe 12.

The residual reaction gas typically contains 0 to 15% molecular chlorine, the balance being mainly hydrogen chloride.

Each column reactor 4, 6 is maintained during the reaction at a temperature in the range 110°—130°C by automatically controlled external cooling means 41, 43 located in pipes 42, 44, respectively.

The hydrocarbons are continuously recirculated through the reactor 4, 6 until a desired degree of chlorination has been attained. Chlorinated product is then discharged from the reactor 4, 6 through the discharge pipes 46, 48 and led away in the common discharge pipe 50 to a storage vessel (not shown).

In the case of paraffin hydrocarbons, in particular, the process of the invention has been found consistently to provide a relatively stable high quality chlorinated product, even from relatively poor quality feedstock. Using mixtures of essentially straight-chain paraffins having an average chain length between 9 and 26 carbon atoms, stable chloroparaffins having chlorine contents of up to 76% by weight may be produced, even from raw material with a higher content of branched-chain paraffinic hydrocarbons than is normally allowable in conventional chlorination processes.

The use of glass column reactors limits the possibility of contamination, and allows effective catalysis of free radical reactions using light sources. Further, the need for mechanical agitation is removed, reducing power requirements. The long residence times associated with stirred reactors are avoided and rapid chlorinations are achieved.

An example of the invention, using the system as illustrated in the attached flowsheet, will now be described.

### Example

Paraffinic hydrocarbon (C14 average) feedstock, of sufficient quantity for a batch in a single column reactor of 450 mm diameter and height of 4 m, i.e. 365 kg, was stripped of free radical reaction inhibitors with $HCl/Cl_2$ reaction gases from the chlorination reactor. The feedstock was passed into a column reactor at a temperature of 70°C. The paraffin was circulated in the reactor and gaseous chlorine addition was started at a rate of about 70 kg/h. The reaction initiation occurred after two minutes, when the chlorine feed rate was increased to 405 kg/h. The temperature in the reactor was allowed to increase to 125°C, and was then maintained at 120—130°C for the duration of the reaction. The pressure in the reactor was maintained at 35—40 kPa above atmospheric. A chlorine content of 52% was attained 115 minutes after initiation, when the chlorine feed was cut off.

The chlorinated product from the reactor consisted of essentially 738 kg of water clear chloroparaffin containing 52% chlorine by weight in two hours of operation, an average output of 363 kg chloroparaffin per hour of operation. The total loss of chlorine from the reactor during the experiment was analysed at 9,5 kg or 2,5% of input chlorine. Losses of chlorine from the stripping column could not be detected. These results must be contrasted with conventional batchwise chlorination processes in agitated reactor vessels where reaction initiation times are of the order of four hours and total chlorination times are of the order of 20 to 30 hours.

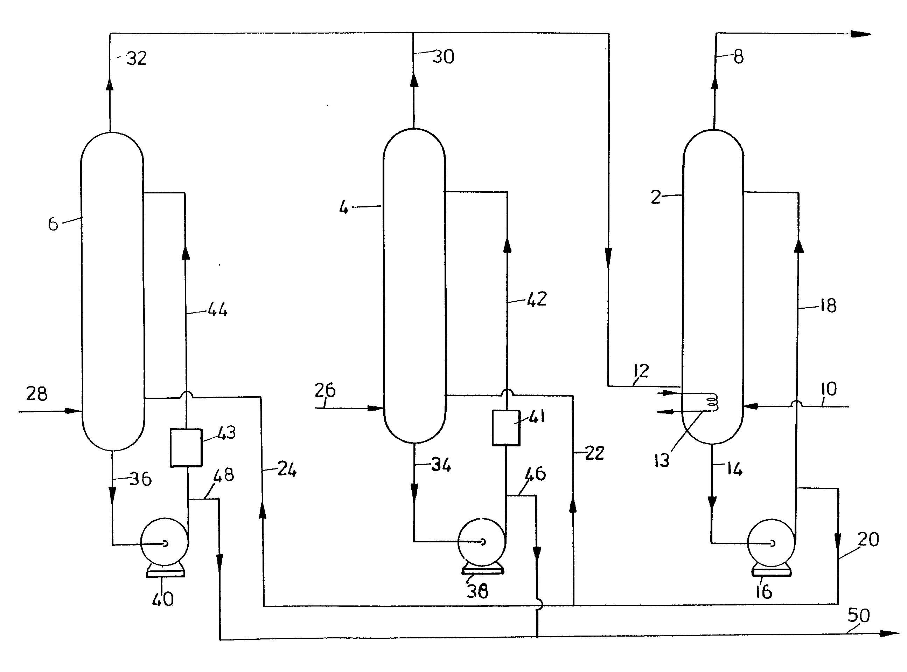

### Claims

1. A batch process for chlorinating a hydrocarbon which is a liquid hydrocarbon or a solid hydrocarbon suspended in a suitable liquid, which comprises:

(a) reacting a charge of the hydrocarbon with chlorine in a vertically disposed column reactor having a reactor volume in which the height is at least four times the longest transverse dimension, to obtain a chlorinated hydrocarbon and residual reaction gases;

(b) stripping a second charge of the hydrocarbon with the residual reaction gases produced in step (a) in order to remove from the second charge inhibitors of chlorine free radical formation;

(c) discharging the chlorinated hydrocarbon produced in step (a) when a predetermined degree of chlorination has been attained; and

(d) recharging the column reactor with the stripped second charge of hydrocarbon produced in step (b).

2. A process according to claim 1, in which, in step (a), the hydrocarbon charge is continuously circulated through the reactor and chlorine gas is continuously supplied to the reactor.

3. A process according to claim 2, in which the hydrocarbon charge and chlorine gas are caused to flow countercurrently, the charge downwardly and the gas upwardly through the reactor, the charge being discharged from a low point of the reactor and recirculated to a high point of the reactor, and the residual reaction gases being removed from a high point of the reactor.

4. A process according to any of claims 1 to 3, in which, in step (b), the second charge and the residual reaction gases are caused to flow countercurrently in a second vertically disposed column reactor having a reactor volume in which the height is at least four times the longest transverse dimension, the second charge downwardly and the gases upwardly through the second reactor, the second charge being discharged from a low point of the second reactor and recirculated to a high point of the second reactor, and residual reaction gases being removed from a high point of the second reactor.

### Revendications

1. Procédé discontinu pour la chloration d'un hydrocarbure qui est un hydrocarbure liquide ou un hydrocarbure solide en suspension dans un liquide approprié, caractérisé en ce qu'il comprend des stades qui consistent à:

(a) faire réagir une charge de l'hydrocarbure avec du chlore dans une colonne réactionnelle verticale ayant un volume réactionnel dont la hauteur est au moins quatre fois égale à la dimension transversale maximale, pour obtenir un hydrocarbure chloré et des gaz réactionnels résiduels:

(b) entraîner une seconde charge de l'hydrocarbure avec les gaz réactionnels résiduels produits dans le stade (a) pour éliminer de la seconde charge les inhibiteurs de la formation du radical chlore libre;

(c) évacuer l'hydrocarbure chloré produit dans le stade (a) lorsqu'un degré prédéterminé de chloration a été atteint; et

(d) recharger la colonne réactionnelle avec la seconde charge d'hydrocarbure entraînée produite dans le stade (b).

2. Procédé selon la revendication 1, caractérisé en ce que dans le stade (a) on fait circuler en continu la charge d'hydrocarbure dans le réacteur et on alimente le réacteur en continu en chlore gazeux.

3. Procédé selon la revendication 2, caractérisé en ce qu'on fait s'écouler à contre-courant la charge d'hydrocarbure et le chlore gazeux, la charge descendant tandis que le gaz s'élève dans le réacteur, la charge étant évacuée en un point bas du réacteur et recyclée en un point haut du réacteur et les gax réactionnels résiduels étant éliminés en un point haut du réacteur.

4. Procédé selon l'une quelconque des revendications 1 à 3, caractérisé en ce que, dans le stade (b), on fait s'écouler à contre-courant la seconde charge et les gaz réactionnels résiduels dans une seconde colonne réactionnelle verticale (42, 44) ayant un volume réactionnel dont la hauteur est au moins quatre fois égale à la dimension transversale maximale, la seconde charge descendant tandis que les gaz s'élèvent dans le second réacteur, la seconde charge étant évacuée en un point bas (34, 36) du second réacteur et recyclée en un point haut (44, 42) du second réacteur et les gaz réactionnels résiduels étant éliminés en un point haut (30, 32) du second réacteur.

## Patentansprüche

1. Diskontinuierliches Verfahren zur Chlorierung von Kohlenwasserstoffen, die als flüssiger Kohlenwasserstoff oder als fester, in einer geeigneten Flüssigkeit suspendierter Kohlenwasserstoff vorliegen, dadurch gekennzeichnet dass man:

(a) eine Charge Kohlenwasserstoff in einem aufrechtstehenden Kolonnenreaktor mit einem Reaktorvolumen, dessen Höhe mindestens das Vierfache seiner längsten Querabmessung beträgt, mit Chlor umsetzt, wobei man einen chlorierten Kohlenwasserstoff und Reaktionsrestgase erhält;

(b) eine zweite Charge Kohlenwasserstoff mit den in Stufe (a) enstandenen Reaktionsrestgasen abstreift, um Stoffe, die die Bildung von Chlorradikalen hemmen, aus der zweiten Charge zu entfernen;

(c) den in Stufe (a) erzeugten chlorierten Kohlenwasserstoff nach Erreichen eines vorbestimmten Chlorierungsgrads austrägt; und

(d) den Kolonnenreaktor mit der in Stufe (b) entstandenen abgestreiften zweiten Kohlenwasserstoffcharge neu beschickt.

2. Verfahren nach Anspruch 1, dadurch gekennzeichnet, dass man in Stufe (a) die Kohlenwasserstoffcharge kontinuierlich im Kreislauf durch den Reaktor führt und kontinuierlich Chlorgas in den Reaktor einleitet.

3. Verfahren nach Anspruch 2, dadurch gekennzeichnet, dass man die Kohlenwasserstoffcharge und das Chlorgas im Gegenstrom, die Charge von oben nach unten und das Gas von unten nach oben, durch den Reaktor strömen lässt, wobei die Charge an einer tiefliegenden Stelle des Reaktors ausgetragen und im Kreislauf zu einer hochliegenden Stelle des Reaktors zurückgeführt wird und die Reaktionsrestgase von einer hochliegenden Stelle des Reaktors abgezogen werden.

4. Verfahren nach einem der Ansprüche 1 bis 3, dadurch gekennzeichnet, dass man in Stufe (b) die zweite Charge und die Reaktionsrestgase im Gegenstrom, die zweite Charge von oben nach unten und die Gase von unten nach oben, durch einen zweiten aufrechtstehenden Kolonnenreaktor mit einem Reaktorvolumen, dessen Höhe mindestens das Vierfache seiner längsten Querabmessung beträgt, strömen lässt, wobei die zweite Charge an einer tiefliegenden Stelle des zweiten Reaktors ausgetragen und im Kreislauf zu einer hochliegenden Stelle des zweiten Reaktors zurückgeführt wird und die Reaktionsrestgase von einer hochliegenden Stelle des zweiten Reaktors abgezogen werden.

0 005 378